# EUROPEAN PATENT APPLICATION

(11) **EP 4 331 546 A1**
(43) Date of publication of application: **06.03.2024**
(21) Application number: 22193026.6
(22) Date of filing: 31.08.2022
(51) Int. Cl.: A61F 13/47, A61F 13/84

(54) **ABSORBENT THERMAL EFFECT PAD**

(71) Applicant: HyPro Innovation GmbH, 71139 Ehningen (DE)
(72) Inventor: WURSTER, Thomas, 71139 Ehningen (DE); SCHWARZ, Niklas, 72074 Tübingen (DE)
(74) Representative: Plischke, Manfred

(57) **Abstract**

The present invention is an absorbent product that provides both a thermal effect, such as cooling, and absorbency for receiving bodily exudates, such as released from the female urogenital tract, whilst providing the thermal effect, such as cooling, for this region.

## Description

### Field of the invention

The present invention relates to an absorbent product that provides both a thermal effect, such as cooling, and absorbency for receiving bodily exudates, such as released from the female urogenital tract, whilst providing the thermal effect, such as cooling for this region.

### Background

Both absorbent pads as well as products proving a thermal effect are well known in the art, as well as products providing both effects simultaneously.

US6916334B2 discloses a thermal pack for application to female breast for therapeutic application of heat or cold to breast, comprises pliable, disk-shaped body having aperture extending approximately through center of body, and including top and bottom walls A common problem experienced by women is discomfort of the vulva, perineum, and surrounding regions. Such discomfort can have a variety of causes, including but not limited to, vaginal itching and burning as a result of infection (e.g., yeast infection), urethral pain or inflammation as a result of infection (e.g., urinary tract infection), vaginal varicosity (e.g., during pregnancy), chronic inflammation (e.g., atopic dermatitis) and in particular strain or even damage to the perineum following child birth.

US 117298260 describes an integral product, wherein a cold pack positioned in the perineal region, overlaying an absorbent pad. Liquid may be distributed towards laterally and longitudinally protruding portions of the pad, and further liquid distribution enhancer aim at further distributing underneath the cool pack.

Following WO2004/067671, the cooling effect may be enhanced by using phase change materials in cool packs.

US5178139 describes an absorbent pad with a thermal pack positioned thereon, whereby the underlying absorbent core establishes a substantial thermal insulation towards the ambient, and an overlying topsheet a lesser one towards the wearer.

US5702375A describes an integrated thermal pack and pad absorbing fluids discharged by patient including flexible liquid impermeable container containing chemical mixture which undergoes thermal reaction between outer and inner sheets.

WO2018017743 discloses a perineal thermal pack with increased fluid containment, wherein a reusable hot or cold pack is positioned on or surrounded by an absorbent pad.

However, it has been discovered that in such designs the liquid handling performance and the cooling or heating effectiveness are not properly adapted, in particular that the geometry of the cooling surface relative to the body does not provide the proper balance of geometrically limited cooling effect versus wearer's comfort as may be impacted by too low temperatures as well as by inadequate fluid handling.

Henceforth, there is a desire to provide a hygiene article that exhibits improved liquid handling performance as well as improved transmission of a thermal effect from the article to a user.

### Summary

The present invention is a disposable absorbent thermal effect pad to be worn in the urogenital region of a wearer, the pad exhibiting a width (y-) extension, corresponding to a left-right orientation on a wearer during use, a longitudinal (x-) extension corresponding to a line reaching from the navel of a wearer through the crotch region to the small of the back, and a thickness (z-) extension perpendicular to both.

The absorbent thermal effect pad comprises
- a topsheet to be positioned towards a wearer during use;
- a thermal effect providing element (TEPE), preferably a cool pack, positioned z-directionally away from a wearer, relative to the topsheet;
- a backsheet positioned opposite of the topsheet, adapted to hinder fluid penetration, preferably being fluid impervious,
- a lower fluid absorbent core, positioned between the topsheet and the backsheet and z-directionally away from a wearer, relative to the topsheet;
- optionally a fluid distribution and thermal insulation (FDTI) layer positioned z-directionally between the topsheet and the TEPE;

The absorbent thermal effect pad further comprises an upper fluid absorbent core, positioned between the TEPE and the topsheet in fluid communication with the lower absorbent core, whereby the upper absorbent core comprises a user-oriented surface comprising at least one opening, preferably a longitudinally extending gap between two lateral portions of the upper absorbent core positioned laterally outwardly of the longitudinal centreline of the absorbent thermal effect pad. Further, the TEPE is positioned between the upper and lower absorbent core and under the at least one opening(s).

In a particular execution, the disposable absorbent thermal effect pad is a cooling pad.

When such disposable absorbent cooling pad comprises an FDTI, wherein the TEPE can be adapted to be cooled to less than 2°C and preferably comprises gel type cooling material.

Preferably, the opening of the upper absorbent core of the disposable absorbent cooling pad, is a longitudinally extending gap between two lateral portions of the upper absorbent core positioned laterally outwardly of the longitudinal centreline of the absorbent cooling pad, such that the temperature in the lateral core region is more about than 2°C, preferably more than about 5 °C, even more preferably more than about 7°C higher than in the gap, when tested according to the temperature measurement test as described herein.

Optionally, the disposable absorbent cooling pad comprises a TEPE of the instant cool pack type, and the pad does not comprise a FDTI.

Optionally, the upper absorbent core may be formed by overfolded portions of a wider lower absorbent core.

A disposable absorbent cooling pad according to the present invention may further comprise one or more of the elements selected from the group consisting of
a) the topsheet is a hydrophobic apertured nonwoven or film material;
b) the absorbent upper and/or lower absorbent core(s) comprise superabsorbent material;
c) the TEPE is adapted for being cooled to less than 15°C and more than about 8°C;
d) the TEPE is adapted for being cooled to less than about 8°C and more than about - 28°C;
e) the upper and lower absorbent cores exhibit a centrifuge absorbent capacity of more than about 10 g, 20 g, 30 g, or even more than 100 g, or more than 200 g, or more than 300 g, and typically less than 1000 g;
f) the TEPE is present at an amount of more than about 20 g, and less than about 300 g. A disposable absorbent thermal effect pad according to the present invention may further comprise one or more additives selected from the group consisting of
g) odour reducing agents, preferably of the cyclodextrin type;
h) perfumes;
i) pain soothing agents.

### Brief description of the Figures

Fig. 1 A to I depict views of executions of an absorbent cooling pad according to the present invention, with Fig. 1A to E show cross-sectional views, Fig. IE a top view with a particular focus on dimensions and Fig. IF to I top views of alternative options for an upper absorbent core.
Fig. 2 depicts schematic temperature profiles over time for comparative products and products according to the present invention.
Fig. 3 depicts a test stand for evaluating the thermal effect of products according to the present invention
Fig. 4 shows test results of products according to the present invention and comparative products.

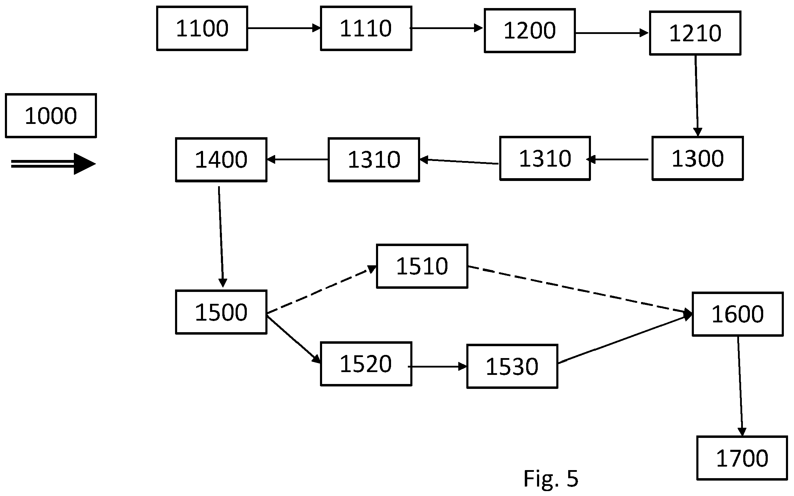

Fig. 5 depicts a flow chart for a process for manufacturing articles according to the present invention.

Same numerals refer to same or equivalent features. Single or multiple apostrophes indicate multiple equivalent features, e.g., "first and second", or "right and left". Drawings are schematic and not to scale.

All percentages are expressed as "weight-%", unless expressly stated.

### Detailed description

The present invention addresses the problem of simultaneously providing
- a thermal effect, such as a cooling effect, to the body of a wearer
- and absorbency of bodily exudates such as wound secretion, breast milk, blood, urine, faeces, menses, or lochia.

This is achieved by use of an absorbent thermal pad comprising a thermal effect providing element (TEPE), also referred to as thermal pack, such as a cool pack, between a first, lower liquid absorbent member positioned away from the wearer and a second, upper absorbent member positioned towards the wearer and in liquid communication with the lower absorbent member. Further, the upper absorbent member is designed such that it does not cover the total user-oriented surface of the pad, but comprises one or more open areas. Thus, in the open areas a more direct thermal effect is experienced, whilst the upper absorbent member, where present, provides a thermal insulation between the thermal pack and the wearer, and consequentially the wearer experiences a stronger cooling effect in the open area(s) and a moderate cooling effect in the area(s) of the upper absorbent member.

This allows to employ thermal packs of a larger size providing - at minimized thickness - a long lasting thermal effect.

Each of the lower and upper absorbent members may be an essentially unitary member or comprise sub-members.

Further, an optional fluid distribution and thermal insulation (FDTI) member, such as a layer, may be positioned between the upper absorbent member(s) and the thermal effect providing material. Apart from improving liquid handling, this provides an even longer lasting thermal effect by allowing to use colder or warmer thermal packs which otherwise would exhibit uncomfortably or even unhealthy temperatures in the skin contacting areas.

The article may be a pad, that is suitable to be applied to the body by a support means, such as underwear, or it may be such a pad integrated into a diaper or pants style article.

In a particular execution, the present invention is a disposable absorbent article particularly suited for women aiming for a thermal effect in the urogenital region, such as upon urogenital pains whilst bodily exudates may be released, such as upon vaginal itching and burning as a result of infection (e.g., yeast infection), urethral pain or inflammation as a result of infection (e.g., urinary tract infection), vaginal varicosity (e.g., during pregnancy), or chronic inflammation (e.g., atopic dermatitis), and in particular after child birth, even accompanied by damage to the perineum.

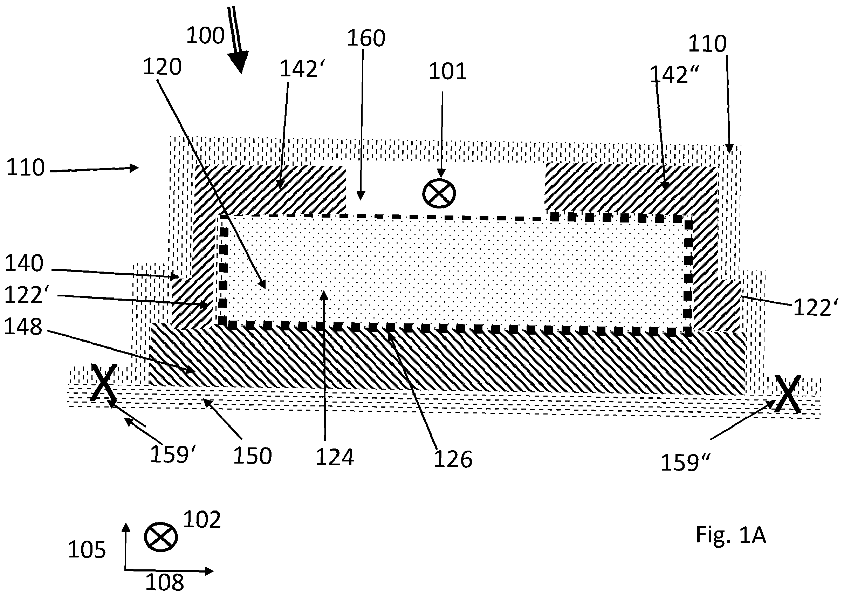

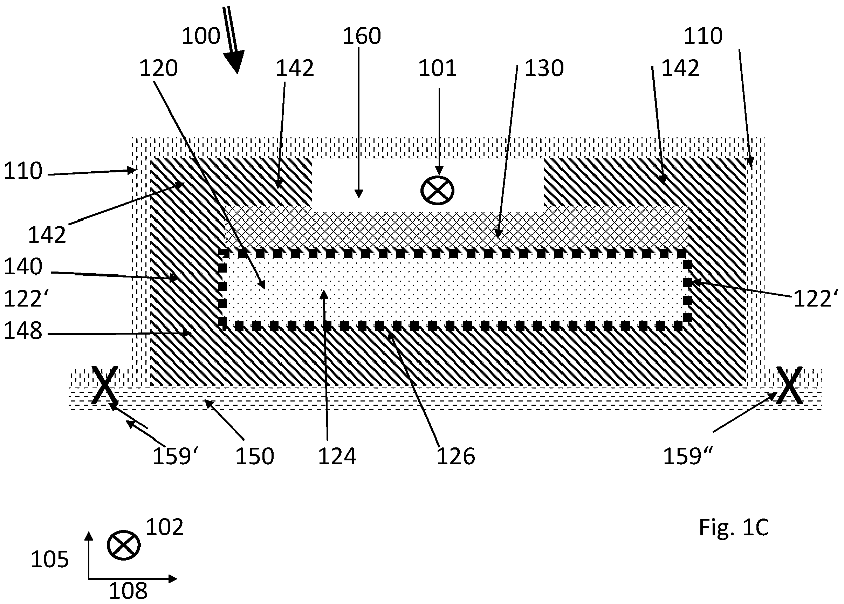

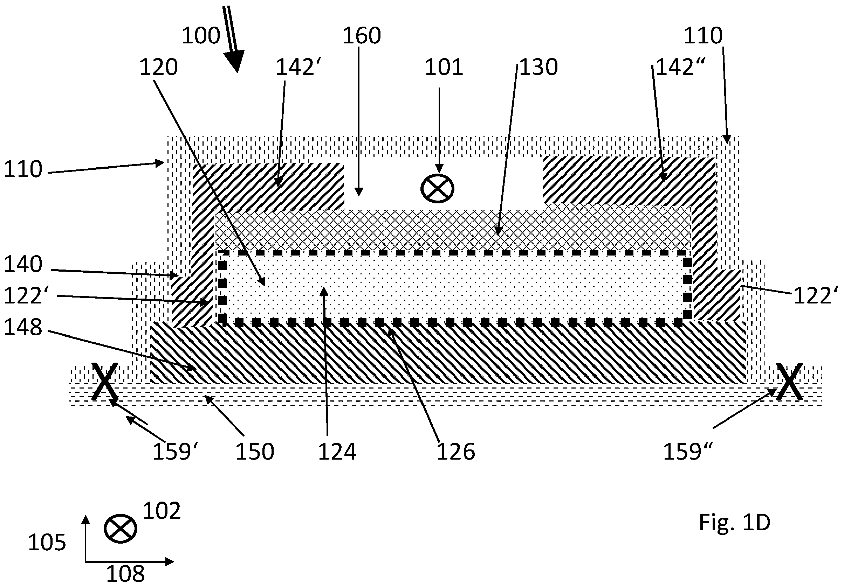

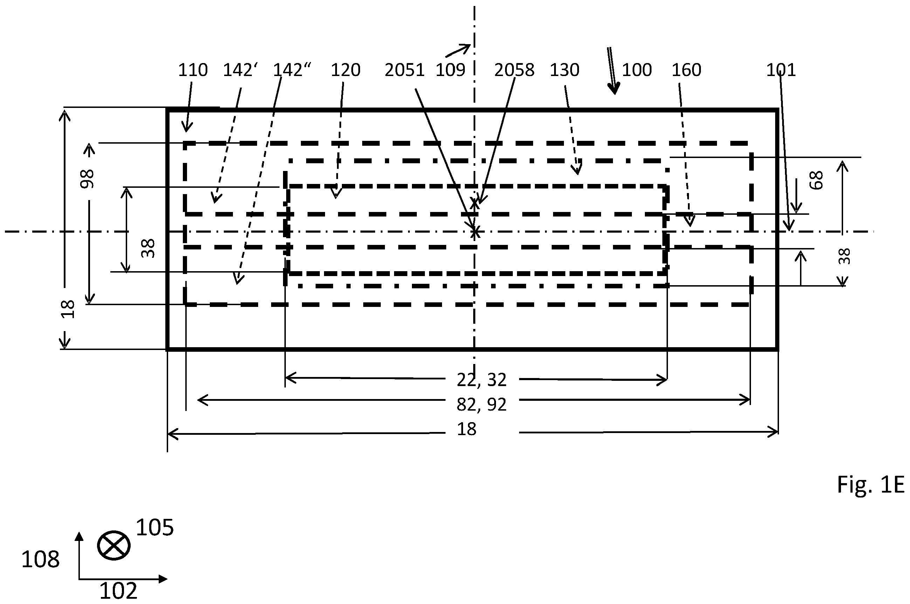

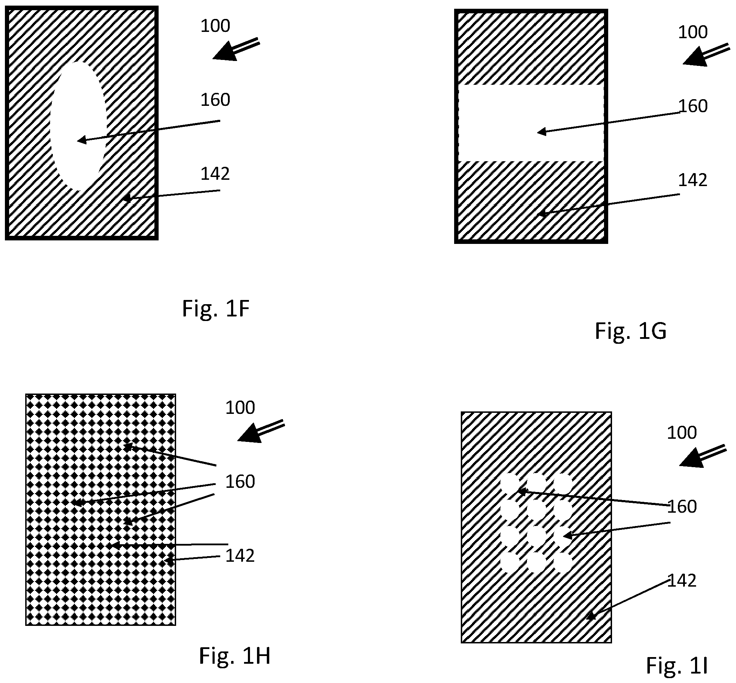

An exemplary execution for an absorbent thermal effect pad 100 as shown in a cross-sectional view in Fig. 1A to ID, comprises
- a topsheet 110 to be positioned towards a wearer during use;
- a thermal effect providing element (TEPE) 120, preferably a cool pack, positioned away from a wearer, relative to the topsheet;
- an optional fluid distribution and thermal insulation (FDTI) layer 130 positioned between the topsheet and the TEPE;
- an absorbent core 140, comprising
- a lower fluid absorbent core 148, positioned away from a wearer, relative to the topsheet and underneath the TEPE; and
- an upper fluid absorbent core 142, positioned underneath the topsheet, i.e., away from a wearer, at least partly overlying the TEPE, and comprising at least an opening 160, preferably a gap along the longitudinal center line of the pad;
- a backsheet 150 adapted to hinder or prevents fluid penetration towards the ambient.

Referring to Cartesian coordinates in Fig. IE, the pad exhibits a length or x-direction 102, a width or y-direction 108, and a thickness or z- direction 105 perpendicular thereto, whereby "up", "upper", "atop" upward" and the like refer to a z-directional position towards the user, and "lower", "downward" and the like refer to the opposite.

The maximum and minimum dimensions should be adapted so as to comfortably fit into the perineal region of a wearer but also to accomplish the required absorbency and cooling effect. Referring to Fig IE,
- the overall length of the pad 12, 52 should be less than about 400 mm, or less than about 350 mm, but more than about 150 mm, and exemplarily be about 320 mm;
- the overall width of the pad 18, 58 should be less than about 150 mm, or less than about 100 mm, but more than about 60 mm, and exemplarily be about 110 mm;
- the length of the absorbent cores 82, 92', 92" should be less than about 380 mm, or less than about 330 mm, but more than about 120 mm, and exemplarily be about 290 mm, whereby the lower and the upper absorbent core preferably, though not necessarily exhibit the same length;
- the width of the lower absorbent core 88 should be less than about 125 mm, or less than about 95 mm, but more than about 45 mm, and exemplarily be about 85 mm;
- the maximum width of the upper absorbent core should match the one of the lower absorbent core and, if executed as two longitudinally extending stripes, each of these should exhibit a width 98', 98" of less than about 55 mm, but more than about 10 mm, and exemplarily be about 35 mm, with a gap width 68 of less than about 30 mm, or less than about 20 mm, but more than about 10 mm, and exemplarily of about 15 mm between the stripes;
- the overall length of the TEPE 22 should be less than the length of the absorbent core 82, 92', 92" and the width of the TEPE 28 should be less than the width of the absorbent core 88, 98', 98", exemplarily with a length of about 205 mm and exemplarily with a width of about 75 mm;
- the FDTI may exhibit the same length 32 as the lower absorbent core 82, at least of the upper absorbent core 92', 92", and a width 38 of less than the width of the TEPE 28, or less than about 90 mm but most preferably more than the gap width 68 between two upper stripes of the absorbent core, if present, and exemplarily may be about 60 mm.

Whilst Fig. 1A to E depict a preferred execution for the upper absorbent pad, namely two parallel longitudinal stripes, leaving a central open area for the stronger cooling effect along the longitudinal center line, other patterns such as exemplarily and non-limiting depicted in Fig IF - 1I, for such an upper absorbent pad are conceivable, and preferred for particular applications.

The pad further comprises a backsheet to prevent penetration of absorbed fluid to the outside, such as outer garments or underwear and a topsheet oriented towards a user and adapted to be in contact with the skin of the wearer.

The backsheet and the topsheet may be connected to each other or may be unitary, with fluid penetration aids like apertures or hydrophilization in the region of the openings, such as the gap. The pad may be adapted to fit to a re-usable chassis, such as mesh underwear or re-usable underwear, or it may form a unity with a chassis so as to allow easy donning and removal.

In a particular execution, the topsheet 110 extends over the gap 160 and laterally over the upper absorbent core 142 of the absorbent core, though a design with the topsheet being extending outside the gap 160 between the upper absorbent core 142 and the TEPE is conceivable.

The topsheet is the layer of the absorbent pad that is destined to be in contact with the wearer's skin. The topsheet is preferably compliant, soft-feeling, and non-irritating to the wearer's skin. Further, at least a portion of the topsheet is liquid permeable, permitting liquids to readily penetrate through its thickness. A suitable topsheet may be manufactured from a wide range of materials, such as porous foams, reticulated foams, apertured plastic films, or woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. If the topsheet includes fibers, the fibers may be spunbond, carded, wet-laid, meltblown, hydroentangled, or otherwise processed as is known in the art, in particular spunbond PP nonwoven.

WO2011/163582 also discloses suitable topsheet having a basis weight of from 12 to 18 g/m² and comprising a plurality of bonded points. Each of the bonded points has a surface area of from 2 mm² to 5 mm² and the cumulated surface area of the plurality of bonded points is from 10 to 25% of the total surface area of the topsheet.

In CN206151698U a natural raw cotton diaper is described, comprising a surface layer, an absorbing layer and an anti-leakage layer, wherein the surface layer is natural raw cotton spun-laced non-woven fabric. The surface layer is provided with multiple openings by mechanical punching, e.g., stamping, rolling. The surface layer retains the original pectin and wax of the raw cotton, thereby exhibiting its natural water-repellent performance.

Kelheim Fibres, Germany, has developed a hydrophobic viscose fibre using plant-based additives, as described in EP2931952 and marketed under the trade designation "Olea", as may be formed into a particularly suitable topsheet e.g., by hydroentangling, and which may comprise apertures to ease penetration of exudates therethrough, see describe generally in e.g., US6632504 (BBA). Further, suitable formed film topsheets are also described in US3929135, or US5006394, or WO2021248436A1, or sold as perforated films by Pantex, IT.

Any portion of the topsheet may be coated with a lotion as is known in the art. Examples of suitable lotions include those described in e.g., US 6716441. The topsheet may also include or be treated with antibacterial agents, some examples of which are disclosed in PCT Publication WO1995/24173. Further, the topsheet, the backsheet or any portion of the topsheet or backsheet may be embossed and/or matte finished to provide a more cloth like appearance.

The backsheet is generally that portion of the absorbent cool pad which forms the majority of the external surface of the article when worn by the user. The backsheet is positioned towards the bottom side of the absorbent core and prevents the exudates absorbed and contained therein from soiling articles such as bedsheets and undergarments. The backsheet is typically impermeable to liquids (e.g., urine). The backsheet may for example be or comprise a thin plastic film such as a thermoplastic film having a thickness of about 0.012 mm to about 0.051 mm. Other suitable backsheet materials may include breathable materials which permit vapors to escape from the article while still preventing exudates from passing through. Exemplary breathable materials may include materials such as woven webs, nonwoven webs, composite materials such as film-coated nonwoven webs, microporous films such as manufactured by Mitsui Toatsu Co., of Japan under the designation ESPOIR NO. Some breathable composite materials are described in greater detail in WO1995/16746 or US5865823.

The backsheet may be joined to the topsheet, and optionally to the absorbent core any attachment means known in the art. For example, the attachment means may include a uniform continuous layer of adhesive, a patterned layer of adhesive, or an array of separate lines, spirals, or spots of adhesive. Suitable attachment means comprises an open pattern network of filaments of adhesive as disclosed in US4573986. Other suitable attachment means include several lines of adhesive filaments which are swirled into a spiral pattern, as is illustrated by the apparatus and methods shown in US3911173. Adhesives which have been found to be satisfactory are manufactured by H. B. Fuller Company of St. Paul, Minn. and marketed as HL-1620 and HL 1358-XZP. Alternatively, and often preferably, the attachment means may comprise heat bonds, pressure bonds, ultrasonic bonds, dynamic mechanical bonds, or any other suitable attachment means or combinations of these attachment means as are known in the art.

Also, the pad may comprise further releasable attachment means for fixing it, e.g., when being worn in underwear.

In a further execution, the pad as described may be permanently connected to a chassis, which refers within the present context to any structure for supporting the pad on the body, as well known from baby and adult incontinence diapers or pants.

It is a particular element of the present invention that a TEPE is positioned between an upper absorbent core 142 and a lower absorbent core 148.

The TEPE 120 may be a conventional heat or cold storage element, comprising the thermally effective material 124 encapsulated in or sandwiched between a TEPE containment material 126. The thermally effective material 124 may be
- a calorific storage element, i.e., exploiting the heat capacity of a material. Considering water as an exemplary thermally effective material, this exhibits a specific heat capacity of about 4.2 kJ/kg/K, when liquid, and about 2.1 kJ/kg/K as ice;
- and/or a phase change material, in the simplest form freezing or melting water, whereby the melting of water requires about 331 kJ/kg;
- and/or an irreversible physico-chemical process, e.g., a chemical reaction or dissolving or concentration change process.

In a particular execution of a cool pack, the TEPE provides a cooling effect versus a body temperature of about 37°C results from body heat being transferred to the colder TEPE. This heat transfer resulting in a true and perceived temperature reduction in the cooled body regions, until the TEPE temperature reaches equilibrium with the body temperature.

Thus, when aiming at a long-lasting cooling effect, conventional systems rely of three effects:
1) Cooling of the TEPE (regardless of comprising PCM or not) to lower temperatures: However, "overcooling" below a lower comfort temperature may cause too big of a temperature difference, which may feel uncomfortable or even hurt by frostbiting below a lower critical temperature.
2) Exploiting reversible PCM effect: PCM are available over a wide range of working temperatures and typically reversible, e.g., paraffin waxes, non-paraffin organics, metallics or salt hydrates, as may be selected according to their properties, specially heat of fusion, latent heat, thermal conductivity, phase change temperature, but also economics, recyclability, but also compatibility with human applications, such as being non-irritating, sufficiently soft, etc.

Typical materials of these categories are freezing or non-freezing gels, such as "gel packs" which are simply pouches of a thickening agent, such as a polymer gel, as may be the same sodium polyacrylate or hydroxypropyl methylcellulose gel used in baby diapers, which can be "frozen" and still remain pliable, preferably combined with a temperature lowering agent, such as n alcohol, such as propylene glycol. A gel pack can usually also be used as a heat pack by microwaving it. 3) The third effect as may be exploited relies on essentially irreversible chemical reactions or mixing effects, and are sometime also referred to as "chemical packs" or - for a cooling effect - "Instant cold packs".

Instant cold packs have two or three compartments separated by breakable barriers. Each of the compartments contain an ingredient which, when mixed with the other ingredient(s), chemically react together or dilute to create a thermal effect. When the cold pack is needed, the user breaks the barriers by bending the pack and squeezing the contents together.

For example, combining ammonium nitrate and water causes a temperature drop to -23°C/-9°F within a few seconds. The temperature remains cold for 20 to 30 minutes. Such pads are not reusable and should be disposed of after use.

Other exemplary material combinations are - without limitation
- the reaction of barium hydroxide octahydrate crystals with dry ammonium chloride
- dissolving ammonium chloride in water
- the reaction of thionyl chloride (SOCh) with cobalt(II) sulfate heptahydrate
- mixing water and ammonium nitrate
- reacting ethanoic acid with sodium carbonate.

However, considering a particularly preferred application of a disposable hygiene product, almost all thereof disqualify at least for cost, handling, and disposability reasons, and the water / ice as well as ammonium nitrate / water system remain the most preferred selections. Also, safety for the use as hygiene article may be a strong advantage thereof. However, it is contemplated, that certain additives can be employed, in particular broadly available and safe salts (like sodium chloride), or gelling agents, such as superabsorbent material

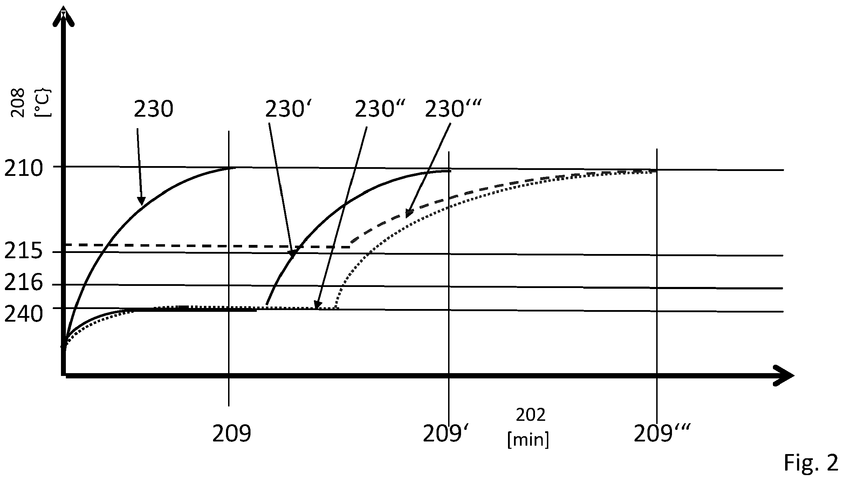

The skilled person will readily realize that the thermal effect also depends on the amount of TEPE in the pad. However, increasing the weight of the TEPE may lead to undesirable wearing discomfort. Typically, the TEPE may exhibit weights of less than about 300 g, or less than about 200 g, or less than about 100 g, and of more than about 20 g, or more than about 30 g, or exemplarily of about 65 g. Further, the TEPE may exhibit average basis weights of more than about 2500 g/m² or more than 3000 g/m² but typically less than about 10000 g/m² and exemplarily about 4200 g/m². Fig. 2, shows schematically the time dependencies of temperatures (200), by plotting temperature 208 in [°C] over time 202 in [minutes] for various executions relative to the body temperature T{Body} 210 as may be assumed - for explanatory purposes only - to be at 37°C. If a cooling element TEPE-1 without a PCM effect is cooled to a lower starting temperature T{0} 220, its surface temperature T{surface} 230 close to the body will then converge towards the body temperature 210, with the exact shape depending primarily on type and amount of material.

If, with otherwise identical conditions, the cooling element TEPE-2 comprises PCM material, and is cooled to the same starting temperature T{0} 220 below the phase change temperature T{PCM} 240, the temperature will increase towards T{PCM} 240 to then remain at that temperature until the phase change is completed, after which the temperature will increase similarly to the one without the TEPE - see curve 230'.

In order to achieve a lasting effect, the T0 and/or T(PCM) should be low. But, for comfort reasons, "overcooling" should be avoided, i.e., the surface temperature should not be below the lower critical temperature T(critical) 216, which is believed to be at about 2°C and preferably at least not for prolonged periods below the lower comfort temperature T(Comfort) 215, which is believed to be at about 7°C. This limits the selection of PCM, reduces the effective time, or requires larger TEPE material volumes.

Thus, apart from exploiting the insulation effect of the upper absorbent core, a further thermal insulating layer, advantageously also providing an improved fluid handling effect as will be discussed in more detail herein below, is positioned between the TEPE and the wearer.

First, this decouples the surface temperature of the pad from the TEPE temperature, allowing to use a wider selection of TEPE materials with lower phase change temperatures without lowering the surface temperature below the comfort temperature, thus avoiding overcooling.

Further, this reduces the heat transfer rate from the wearer to the TEPE, thereby slowing the heating of the TEPE resulting in a longer lasting cooling effect.

Referring to Fig. 2 again, the temperature curve for the TEPE material is similar to the curve 230' as shown before, but - due to the reduced heat transfer - the temperature remains longer at the phase change temperature plateau 230, to then raise at a slower rate. Even more so, the surface temperature, indicated by curve 250 stays above the comfort temperature 215, allowing a more moderate but longer lasting cooling effect.

The thermal properties of such a thermal insulating material can be described by the heat conductivity Lambda [mW/(m^{∗}K)] of the dry material, as may be determined according to ISO 8301, and may be less than about 2000, or less than about 500, or less than about 200, or less than about 100, or less than about 50, or less than about 40, and typically more than about 25, all expressed in units of [mW/(m^{∗}K)].

A pad according to the present invention further provides a liquid absorbency functionality for exudates from the urogenital tract of a wearer, typically a female wearer, and thus comprises an absorbent core. The principle of an absorbent core suitable for the present invention is well-known from absorbent articles, especially from feminine hygiene articles, as urinary incontinence article, menstrual products, or post-partum articles.

The absorbent layer may include absorbent polymer particles, also referred to as superabsorbent polymeric (SAP) material, alone or in combination with other materials, such as cellulose fiber. The absorbent polymer particles may be immobilized on or in a substrate layer by various known approaches, such as without limitation:
- an adhesive material, see e.g., US8919407A1(P&G, Blessing) describing a thermoplastic adhesive material;
- or hydrogen bonds in combination with compression, see e.g., EP2123440A1 (McAirlaid);
- or more preferably by mechanical entanglement, as may be further enhanced by thermal or ultrasonic bonding, see e.g., WO2014/001487 (C4S).

The absorbent polymer particles may be spherical, spherical-like, ellipsoid, or irregularly shaped, such as ovoid-shaped particles of the kind that may be obtained from inverse phase suspension polymerizations. The particles may, optionally, be agglomerated at least to some extent to form larger irregular agglomerations of particles. The absorbent polymer particles may be selected from among polyacrylates and polyacrylate based materials that are internally and/or surface cross-linked, such as for example partially neutralized cross-linked polyacrylates or acid polyacrylate. Examples of absorbent polymer particles suitable in the present disclosure are described for instance in the PCT Pat. App. Nos. WO07/047598, WO07/046052, WO2009/155265 and WO2009/155264.

The substrate layer of the absorbent core structure may be any material capable of supporting the absorbent polymer particles. It may be a web or sheet material, such as foam, film, woven and/or nonwoven material.

Nonwoven materials and processes for making them are generally known in the art. Generally, processes for making nonwoven materials include two steps: depositing and accumulating fibers to the desired basis weight onto a forming surface, and consolidating and bonding the accumulated fibers to form a coherent web. The first step may include spunlaying, meltblowing, carding, airlaying, wetlaying, coforming and combinations thereof. The bonding step may include hydroentanglement, cold calendering, hot calendering, through air thermal bonding, chemical bonding, needle punching, and combinations thereof.

Yet another variant for an absorbent core may be along the teachings of Polygreen Ltd, IL, see EP3773388A1 (Polygreen), disclosing a textile composite article comprising textile fibers and polymers network having natural polymer crosslinked to synthetic polymer or WO2021168378A1, disclosing a multilayer absorbent product comprising a layer of knitted textile material or textured plastic yarn comprising deposits of natural polymer crosslinked to synthetic polymer, covering fibers or pore walls underneath the deposits. In a variant of these disclosures, the deposits may also be applied in-situ, i.e., along the overall pad manufacturing process.

An absorbent core according to the present invention comprises an upper absorbent core, oriented towards the user, and a lower absorbent core opposite thereof, with the TEPE sandwiched there between.

In a first execution, exemplarily, but not limiting depicted in Fig. 1A, the absorbent core 140 is made from a wider layer of absorbent material 145, which is folded around the TEPE 120 along the longitudinally extending side margins 122', 122" of the TEPE, such that a gap is formed between the overfolded edges of the absorbent material forming strips of the upper absorbent core 142.

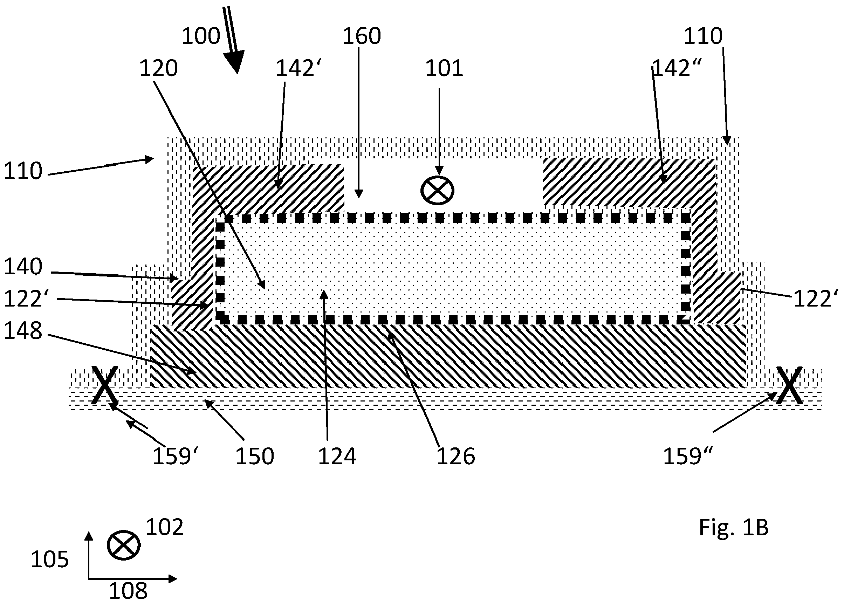

In a second execution, exemplarily, but not limiting depicted Fig. 1B, the absorbent core 140 is made from strips of absorbent material, with the lower absorbent core absorbent core 148 as a wider strip underlying the TEPE 120 and the upper absorbent core as two narrower parallel strips 142', 142" overlaying the TEPE 120, which are positioned essentially parallel to the longitudinal center and distanced apart by the gap width 68 of the center gap 160. In the execution as depicted in Fig. 2B, the upper absorbent core portions extend laterally outwardly of side margins 122' and 122" of the TEPE and are connected in a fluid communicating manner to the lower absorbent core.

Whilst the upper and lower absorbent cores may be of the same type and be essentially identical in composition, it is also contemplated that the upper and the lower cores vary in composition, thickness, absorbency, or other properties. For example, the lower absorbent core may be made as a pre-manufactured absorbent web material, whilst the upper absorbent cores are different strips of web materials, or may even be applied in-situ, e.g., by applying the above referenced Polygreen technology.

In either of these executions, the upper absorbent core does not completely cover the TEPE but comprises at least one opening therethrough, here shown in a preferred execution as a gap 160 extending along the longitudinal center line 101 of the article, where there is essentially no absorbent core positioned between the TEPE and the user-oriented surface of the article. The opening(s) respectively this gap are/is adapted to be positioned in the perineal region of the wearer and allows liquids to penetrate through.

The gap 160 extends longitudinally at least over the length of the TEPE, and may have the overall length of the article. The gap should exhibit a cross-directional gap width 68 of more than about 10 mm, or more than about 13 mm, or more than about 15 mm, and of less than about 25 mm, or less than about 21 mm, or less than about 18 mm, with a particularly well-functioning width of about 16 mm.

It should be noted, that the upper absorbent core also provides an increased insulation effect towards the user, as the absorbent material functions as an additional spacer and heat sink between the TEPE and the body.

In any of the executions of the absorbent core, e.g., as described the overfolded or the upper strips variant, the liquid as received in the gap should be further distributed towards other regions of the absorbent core.

In a first execution of the upper absorbent cores being overfolded there is direct fluid connection between the upper and the lower absorbent core s along the overfolded portions.

In a second execution of the two parallel strips forming the upper portions 142' and 142" of the upper absorbent core, the fluid communication between the upper and lower absorbent cores may be achieved by appropriately connecting these laterally outside of the TEPE by connections 149', 149", respectively.

For either of these executions, the liquid communication may also be achieved by providing liquid communicating openings through the TEPE, which, however may be less preferred from a manufacturing process point of view.

However, even though absorbent cores may exhibit x-y-directional liquid distribution capability, it is highly preferred that the upper absorbent cores 142 are prevented from being overloaded relative to their total liquid capacity, and fluid is more readily transmitted to the lower absorbent core.

It is well known from the design of absorbent articles that such a functionality can be achieved by liquid distribution materials, also referred as "acquisition and distribution materials",

Thus, it is a particularly preferred element of the present invention that a material combining the fluid distribution and the thermal insulation (FDTI) functionality may be positioned in the gap region and extends laterally outwardly, such that is z-directionally between the portions of the upper absorbent core and the TEPE.

The material may have the form of, e.g., a layer, mat or other body formed of or including, e.g., comminuted cellulose fibers, or other hydrophilic natural, semi-synthetic or synthetic fibers or other material that may be used to form a mat, layer or other body.

The FDTI materials may be made of a nonwoven material based on synthetic or cellulosic fibers and having a relatively low density, which may typically be more than about 0.03 g/cm³ or more than about 0.05 g/m³ or less than about 0.25 g/cm³, or less than about 0.15 g/cm³, all measured at 0.30 psi (2.07 kPa). The FDTI may typically have an average basis weight of more than about 20g/m² or more than about 30 g/m² or more than about 40 g/m² or more than about 100 g/m², and typically less than about 300 g/m², or less than about 200 g/m² or less than about 150 g/m².

The FDTI may include a non-woven, which may be hydrophilic and which may comprise natural or synthetic polymeric fibers, such as high surface area fibers, thermoplastic binding fibers, polyethylene fibers, polypropylene fibers, PET fibers, rayon fibers, lyocell fibers, *eucalyptus* fibers and mixtures thereof, as may have been formed by spunmelting, carding or other conventional methods and may be bonded by through-air, resin addition, or embossing techniques. Further, according to a certain example, the FDTI may include the chemically cross-linked cellulosic fibers, as may be crimped, twisted, or curled, or a combination thereof including crimped, twisted, and curled, as described e.g., in US 2008/0312622 A1 as part of an acquisition system.

Also collapsible, hydrophilic, flexible, nonionic polymer foam for absorbent hygiene articles reexpands with absorption of fluids, such as described in e.g., EP067339A1.

Particularly preferred FDTI materials are open structured nonwoven webs with polyester fibers, as may be a single or copolyester or bicomponent fibers thereof, of a thickness of more than about 2 dTex or more than about 3 dTex, as may be powder bonded air through bonded exhibiting a basis weight of between 35 and 50 g/m², such as available from General Nonwoven, Turkey, under the designation GA0110 to GA0112, or ParaTherm Loft 161 of TWE Group, Germany, or STA CTCT 50 of Shalag Ind. Ltd., Israel..

### Exemplary execution

Exemplary executions of a pad according to the present invention has been prepared

### Example A

First, a TEPE was made, using a commercially available pad from GELLO^{®} GmbH Geltechnik, Germany, under the designation "Hot and cold compress", with a width of 7.5 cm and a length of 35 cm, which was modified by shortening it to a length of 20 cm, adjusting the gel weight to 62 g, i.e., a total TEPE weight of about 65 g, and re-closing the cut opening by ultrasonic welding. Further, a gel free perimeter was formed by squeezing the from the longitudinal margins towards the center and fixing the perimeter by ultrasonic welding.

The gel pad was positioned centred on a multibonded airlaid absorbent material, available from Main S.p.a., Italy, under the designation Softcare HAP-33-55%SAP, comprising fluff pulp, EVA copolymer. This was overfolded around the longitudinal margins of the TEPE, forming two lateral upper core stripes at about 35 mm.

As FDTI, an air-through bonded polyester web was inserted between the upper absorbent core stripes and the TEPE, available from Shalag Nonwovens Ind. Ltd, Israel, under the designation STA CTCT 50, exhibiting a basis weight of 50 g/m², and length and width corresponding to the dimensions of the TEPE, i.e., 7.5 cm by 20 cm.

The composite was enveloped by a "textile backsheet" material, as available from Hüner Nano Textile Plastic Co. Turkey, at 25 g/m², comprising 50% of a polyethylene film, 45.5 % of polypropylene nonwoven and 4.5 % of adhesive, and a hydrophobic apertured nonwoven of 35 g/m², available e.g., from Pelz Group, Germany, under the designation Sawasoft 50FC050903. The envelope materials were connected by ultrasonic bonding

### Comparative example

As a comparative example, a cooling pad was used, which was commercially available under the designation "Instant Perineal Cold Packs" from First Days Maternity Supplies Ltd, UK, exhibiting a total weight of about 138 g. It comprises an activatable cool pack with a weight of about 108 g believed to comprise ammonium nitrate and water, which are to be activated directly prior to use by squeezing the plastic envelope. The product further comprises an absorbent core based on fluff pulp and superabsorbent wrapped with tissue. The cool pack is positioned centered thereon (i.e., towards the wearer) leaving the core to project about 7 mm on the longitudinal sides. Further, the pad comprises conventional polypropylene nonwoven wrap around with an overlap bonded by hotmelt on the opposite side of the wearer. In addition, the product comprises a strip of hotmelt covered by silicon paper along the overlap of the wrap dedicated to fix the product in wearer's underwear. Such product was produced by INTCO Medical (HK) Co. Ltd, Hong Kong and distributed by First Days Maternity Supplies Ltd., UK, Ref. PER613, lot 021TCC17006.

### Example B

The second example was prepared as Example A, except for omitting the FDTI and employing the cool pad of the comparative example.

### Test results

Following the test procedure as described in below, the sample pads were cooled in a refrigerator or a freezer, and profiles of surface temperatures over time were measured under laboratory conditions.

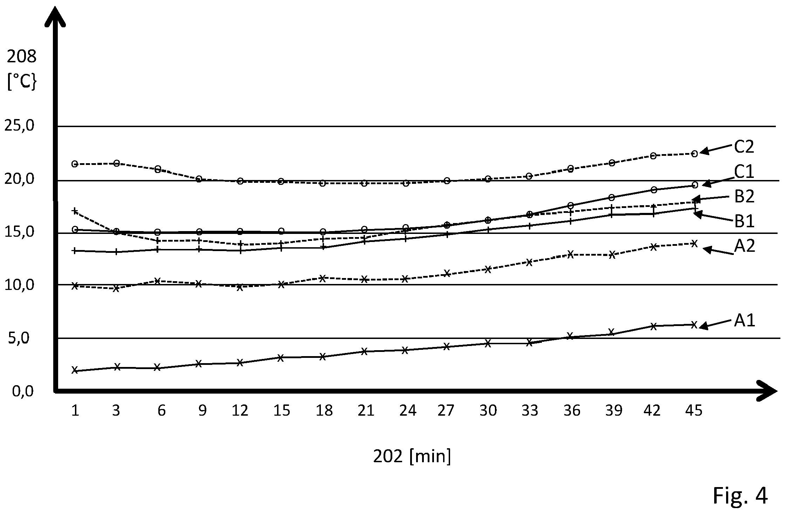

Fig. 4 depicts the time dependencies of the temperatures, wherein the letter denotes the test specimen (examples A and B and comparative pad C) and the suffix number the measurement points 1 (gap region) and 2 (laterally offset).

As can be seen, Example A exhibits the lowest temperatures A1 in the gap region, but well above the initial TEPE temperature of about -18°C and above the lower critical comfort temperature of 2°C. This level may further be adjusted by enhancing the FDTI material, but preferably not to the extent of an upper core, although even this temperature is below the temperatures C1 and C2 of the comparative example, whereby the initial dip in temperatures of C2 may be attributed to uneven activation of the instant cool pad of comparative pad C.

The measurements for B2 indicate that without a FDTI an instant cool pack picks up less energy via the lateral portions and hence allows longer wearing times with lower temperatures in the gap region.

### Product Test methods

### Pad temperature measurements

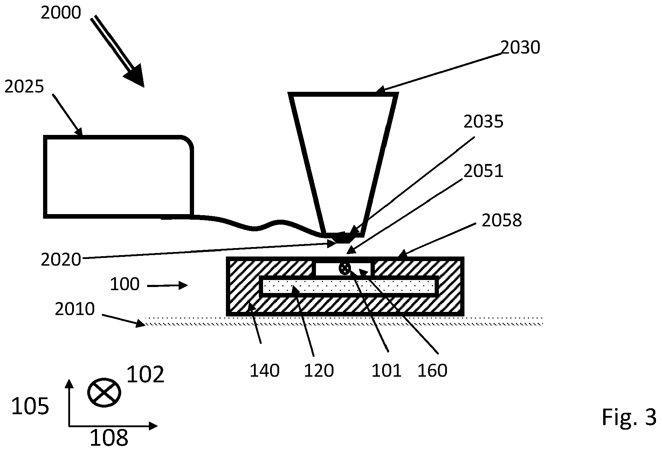

A test-stand as set up according to Fig 3 comprises
- a flat sample support surface 2010;
- a temperature probe 2020 connected to a temperature indicator or recorder 2015, Voltcraft K101 digital Thermometer, or equivalent, attached to a circular polypropylene plate of a pressure weight,
- a pressure weight exerting 4.8 kN/m² over a diameter of 2.8 cm, corresponding to an area of 6.15 cm² by a 300 g weight, executed as a water reservoir (standard polyester water bottle) at room temperature, with approximately 0.25 1 tap water, hold in the vertical by a support frame, or equivalent.

### Sample preparation:

Cool pads: The samples have been conditioned for at least 2 hours
   a) a refrigerator at about 8 °C
   b) a freezer at about -18 °C
Instant pads: conditioned at room temperature

The samples have been taken out and put onto the test stand at standard conditions and a first and a second measurement points were identified corresponding to the pudendal region during use, wherein the first point 2051 (see Fig. 3) was positioned cross-directionally centred, i.e. in the gap region, and the second point 2058 (see Fig. 3) was positioned on one of the upper core regions, longitudinally and cross-directionally centred thereon, but in any case so that it is positioned z-directionally over the TEPE. If nor upper core is present, the second point is positioned half way from the longitudinal center line to the longitudinally extending side margin.

Temperature is recorded manually or automatically over 45 min, with a reading at least every 2 minutes.

All measurements shall be executed with at least two replicates.

### Centrifuge Capacity Test

This test is generally following the Edana NWSP 241.0.R2 (15) Polyacrylate Superabsorbent Powders - Determination of the Fluid Retention Capacity in Saline Solution by Gravimetric Measurement Following Centrifugation, but applying it to an absorbent article, as per Test Method PA07/05 of Hygiene-Technologie GmbH (Hy-Tec), Haan, Germany. To this end, a test sample as treated according to the free swell absorption test is positioned within a commercial centrifuge exerting a centrifugal force of about 250 times gravity (e.g., by having an internal diameter of 225 mm at a rotation of 1400 rpm) for 3 mins. The test specimen is removed and weighed and the centrifuge absorbent capacity recorded in grams.
Basis weight: "Mass per unit area", EDANA NWSP 130.1 R0 (15).
Thickness: "Standard Thickness determination" - EDANA NWSP 120.6.R0 (15), Option A.

Pads according to the present invention may suitably be produced by the following manufacturing process steps:
- Positioning two or more webs or material pieces relative to each other and to the equipment sections;
- Combining or connecting the positioned webs or material pieces temporarily or permanently, e.g., by thermal or ultrasonic melt-fusion bonding, or glue application;
- Separating, e.g., an essentially continuous web into web pieces, or slitting portions of a web or material piece, as may be a straight lit or contour cutting,
The process may be executed such that sections of the web are fed into respective equipment sections arranged along the path of a web material, each section being adapted to perform the predetermined process step, by comprising
- Positioning tools, e.g., robots or removal tools for removing a material piece from a stack, but also transfer or guide rolls, vacuum suction boxes, etc.
- Connecting tools, e.g., thermal or ultrasonic melt-fusion bonding units, or glue applicators;
- Separation tools, e.g., slitter or cutter units;

Thus, referring to Fig. 4 depicting the process flow chart of the manufacturing method 1000, the manufacturing method 1000 comprises the steps of
- providing
   continuous supply units for
      webs of topsheet (110) and backsheet (150);
      FDTI material (130)
   a TEPE / CoolPack (120) supply unit for providing a TEPEs from
      - a continuous TEPE material supply in a web form, the TEPE supply unit comprises
         - an unwind or deboxing stand,
         - web guide units, as guide bars and driven or idle roller bars,
         - a separation unit for separating a series of individualized absorbent cores from the continuous web;
         or
      - an individualized TEPE supply unit for delivering preformed TEPE, comprising
         - a supply of boxed, stacked or otherwise arranged individualized TEPE;
         - a TEPE feeding unit, such as a positioning robot or a rotary or longitudinal feed system with attachment units, such a vacuum;
   an absorbent core supply unit, for providing absorbent cores from
      - a continuous core material supply in a web form, the absorbent core supply unit comprises
         - an unwind or deboxing stand,
         - web guide units, as guide bars and driven or idle roller bars,
         - a separation unit for separating a series of individualized absorbent cores from the continuous web;
         or
      - an individualized absorbent core supply unit for delivering preformed absorbent cores ("patches"), comprising
         - a supply of boxed, stacked or otherwise arranged individualized cores;
         - an absorbent core feeding unit, such as a positioning robot or a rotary or longitudinal feed system with attachment units, such a vacuum;
      movable support and transfer unit optionally comprising sub-units, comprising a plurality of treatment sections along the direction of movement (machine direction), adapted to be operated in a step-by-step movement;
   cutting & connection means;
- supplying a continuous web of backsheet material (1100);
- positioning a predetermined length of said backsheet on a first treatment section of said support unit (1110);
- providing a piece of absorbent core from the absorbent core supply unit (1200);
- positioning an absorbent core in the first section of the support unit on said length of the backsheet at a predetermined position (1210);
- providing a piece of TEPE (1300);
- positioning the TEPE on the absorbent core (1310);
- positioning a predetermined length of FDTI material on the TEPE (1400);
- positioning the upper absorbent core over the FDTI, thereby forming an interim composite (1500) by
   - overfolding a wider portion of the lower absorbent core laterally extending outwardly relative to the TEPE such that a portion thereof overlays the TEPE, thus forming the upper absorbent core (1510),
   or
   - positioning separate portions of the upper absorbent core on top of the TEPE (1520); to then
   - connect laterally outwardly protruding portions of the upper and lower absorbent core to each other (1530);
- providing topsheet web material (1600);
- positioning the topsheet web onto the composite in registry with the backsheet (1610);
- connecting the topsheet and the backsheet (1620);
- separating the combined topsheet and backsheet to form separated individual article (1630).

Whilst such a process can be quite readily executed on a conventional high speed continuous manufacturing line, another execution can be a clocked manufacturing process allowing easy manufacturing of such pads on simple manufacturing equipment, thereby avoiding complex machinery and process control as required by conventional continuous high speed manufacturing process as well-known for the "fast moving consumer goods".

Within the present context, the term "clocked" refers to a production process, where several process steps are executed consecutively on an essentially continuous web in manufacturing equipment sections at an essentially zero or low processing speed of the web.

Notwithstanding these consecutive process steps, further process steps may be executed when transferring the continuous web(s) from one process section to the following section at a higher transfer web speed.

## Claims

1. A disposable absorbent thermal effect pad to be worn in the urogenital region of a wearer,
said pad exhibiting
a width (y-) extension, corresponding to a left-right orientation on a wearer during use;
a longitudinal (x-) extension corresponding to a line reaching from the navel of a wearer through the crotch region to the small of the back;
a thickness (z-) extension perpendicular to both;
comprising
- a topsheet to be positioned towards a wearer during use;
- a thermal effect providing element (TEPE), preferably a cool pack, positioned z-directionally away from a wearer, relative to the topsheet;
- a backsheet positioned opposite of said topsheet, adapted to hinder fluid penetration, preferably being fluid impervious,
- a lower fluid absorbent core, positioned between said topsheet and said backsheet and z-directionally away from a wearer, relative to said topsheet;
- optionally a fluid distribution and thermal insulation (FDTI) layer positioned z-directionally between said topsheet and said TEPE;
**characterized in that**
- it further comprises an upper fluid absorbent core, positioned between said TEPE and said topsheet in fluid communication with said lower absorbent core;
- said upper absorbent core comprises a user-oriented surface comprising at least one opening, preferably a longitudinally extending gap between two lateral portions of said upper absorbent core positioned laterally outwardly of the longitudinal centreline of said absorbent thermal effect pad;
- said TEPE is positioned between said upper and lower absorbent core and under said at least one opening(s).

2. A disposable absorbent thermal effect pad according to claim 1, wherein said thermal absorbent effect pad is a cooling pad.

3. A disposable absorbent cooling pad according to claim 2 comprising an FDTI, wherein said TEPE is adapted to be cooled to less than 2°C and preferably comprises gel type cooling material.

4. A disposable absorbent cooling pad according to claim 2 or 3, whereby said opening of said upper absorbent core is a longitudinally extending gap between two lateral portions of said upper absorbent core positioned laterally outwardly of the longitudinal centreline of said absorbent thermal effect pad,
and the temperature in the lateral core region is more about than 2°C, preferably more than about 5 °C, even more preferably more than about 7°C higher than in the gap, when tested according to the temperature measurement test as described herein.

5. A disposable absorbent cooling pad according to any of claims 1 or 2, wherein the TEPE is of the instant cool pack type, and the pad does not comprise a FDTI.

6. A disposable absorbent thermal pad according to any of the preceding claims, wherein the upper absorbent core is formed by overfolded portions of a wider lower absorbent core.

7. A disposable absorbent cooling pad according to any of the preceding claims, comprising one or more of the elements selected from the group consisting of
a) said topsheet is a hydrophobic apertured nonwoven or film material;
b) said absorbent upper and/or lower absorbent core(s) comprise superabsorbent material;
c) said TEPE is adapted for being cooled to less than 15°C and more than about 8°C;
d) said TEPE is adapted for being cooled to less than about 8°C and more than about - 28°C;
e) said upper and lower absorbent cores exhibit a centrifuge absorbent capacity of more than about 10 g and preferably less than about 1000 g;
f) said TEPE is present at an amount of more than about 20 g, and less than about 300 g.

8. A disposable absorbent thermal effect pad according to any of the preceding claims, further comprising one or more additives selected from the group consisting of
g) odour reducing agents, preferably of the cyclodextrin type;
h) perfumes;
i) pain soothing agents.
